# EUROPEAN PATENT SPECIFICATION

(11) **EP 0 384 547 B1**
(45) Date of publication and mention of the grant of the patent: **18.10.1995**
(21) Application number: 90200866.3
(22) Date of filing: 22.04.1986
(51) Int. Cl.: A23L 3/3472, C11B 5/00, A23C 15/20, A23D 7/06, A23D 9/06, C09K 15/34

(54) **Oxidation-resistant alimentary fatty compositions containing an active oxidation-preventing agent extracted from vegetable tissues**
Oxidationswiderstandsfähige, essbare Fettzusammensetzungen, die ein aus pflanzlichem Gewebe extrahiertes, aktives Oxidation verhinderndes Mittel enthalten
Compositions de graisses alimentaires, résistant à l'oxydation et contenant un agent anti-oxydant actif, extraites de tissus végétaux

(30) Priority: 24.04.1985 US 726540
(43) Date of publication of application: 29.08.1990
(62) Divisional of application: 86200675.6
(73) Proprietor: BAR-ILAN UNIVERSITY, IL-52 100 Ramat Gan (IL)
(72) Inventor: Albeck, Michael, Ramat Gan, IL-52223 (IL); Grossman, Shlomo, Ramat Gan, IL-52444 (IL)
(74) Representative: Fusina, Gerolamo

(56) References cited:
- US-A- 2 098 254
- US-A- 2 198 203
- US-A- 3 850 907
- US-A- 4 011 206
- CHEMICAL ABSTRACTS, vol. 91, no. 15, 8th October 1979, page 495, abstract no. 122353s, Columbus, Ohio, US; Y. NAKAMURA et al.: "Fractionation of antioxidative constituents of "Okara"", & KAGOSHIMA DAIGAKU KYOIKUGAKAKU KENKYU KIYO, SHIZEN KAGAKU HEN 1978 (PUB. 1979). 30, 41-9
- FOOD SCIENCE & TECHNOLOGY ABSTRACTS, 1983, no. 83-07-T0402 (83042100); S. NAITO et al.: "Studies on natural antioxidant. III. Fractionation of antioxidant extracted from garlic", & JOURNAL OF JAPANESE SOCIETY OF FOOD SCIENCE AND TECHNOLOGY NIPPON SHOKUHIN KOGYO GAKKAISHI, 28(9), 1981, 465-70
- FETTE, SEIFEN, ANSTRICHMITTEL, vol. 71, no. 7, 1969, pages 537-542, Hamburg, DE; H.P. KAUFMANN et al.: "Pro- und Antioxydantien auf dem Fettgebiet XXV: Über ihre Lokalisierung in Samen und Früchten sowie in Kartoffeln"

## Description

This application is a divisional of EP-A-0 201 956, published on the 20.11.1986, Bulletin 86/47.

This invention relates to water-soluble antioxidants adapted to prevent the oxidation of alimentary fatty substances.

Many of the antioxidants used for preventing the degradative oxidation of fatty substances are long since known in the art and examples of synthetic materials are BHA (butylated hydroxyanisole), BHT (butylated hydroxy toluene), propyl gallate and alpha-tocoferol, to mention but a few.

On the other hand, it is known that certain plant tissues contain natural antioxidants.

Certain of these antioxidants have been obtained in crude form and have been shown to have an effect on commercial soybean enzyme lypoxygenase (J.Food Science, 36, 571 (1971).

JP-A-58-4286 discloses an alkali-organic solvent extracting process which obtains an antioxidant from powdered white pepper.

In Medycyna Weterynaryina, 28, 430-433, a procedure is disclosed for extracting dried hay or Urtica with boiling distilled water: this product was used within 48 hours of its preparation as an antioxidant for fish meal.

The applicants have discovered that antioxidants obtained by water extraction of aërial plant tissues can be profitably used for the preservation of alimentary fatty substances in place of conventional antioxidants such as the aforementioned BHT, BHA and the like.

The invention, therefore, provides an oxidation-resistant alimentary fatty composition comprising an alimentary fatty substance, and an active fat-oxidation preventing agent, obtained from vegetable aerial tissues of a plant selected from the genera Spinacia, Trifolium, Medicago, Nicotiana, Alga, Allium, Zea and Penicillaria, characterised in that the active agent is at least one of the coloured antioxidant active fractions which are chromatographically separated, by purification with dextran cross-linked with epichlorohydrin and having a pore size of from 40 µm to 150 µm as the chromatographic column packing, from an aqueous vegetable extract obtained at a temperature of from 4°C to 100°C, preferably at 25°C, by comminuting in the presence of water, of said vegetable tissues.

The plants which can be used as a source of the water-soluble extract include the aërial plant tissues, such as stems and green leaves, of the genera aforementioned.

The antioxidant effect is determined by the well known TBA (thiobarbituric acid) test: the latter is described, for example, in Food Res., 23, 620 (1958).

The water-soluble antioxidant can be extracted from the plant tissues using a plant-to-water ratio of from 0,5:100 to 1,0:0,5 in terms of weight:volume, such as g/ml.

The ratio of 2:1, as defined above, is preferably used when treating comminuted plant material. The comminution can be carried out at a temperature of from 4°C to 100°C, preferably at 25°C, using any appropriate comminuting apparatus, such as blenders, grinders and the like.

The extracted plant material is discarded by filtration, centrifugation, decantation, froth flotation and, in general, by any technique suitable for separating solids from liquids.

The crude antioxidant may be used in the dilute form, as an aqueous extract, or as a purified extract: the latter is the preferred form, and is obtained by evaporation or lyophilization (freeze drying) in order to obtain a dry, water-soluble antioxidant, to be subsequently fractionated and purified chromatographically.

As a rule, the raw antioxidant powder is dissolved in water to prepare a solution, containing from 10% to 30% by weight of the powder, on a weight:weight basis.

The solution so prepared is poured on the top of the chromatographic column and is allowed to move therethrough, as usual.

The various fractions are eluted using water as the eluant, and the various fractions are separately collected.

The individual fractions can be further purified by repeating the chromatographic extraction process: in this case, the column-packing medium should have a smaller pore-size.

Sephadex G-25 (Reg.Trade Mark), which is a medium-grade chromatographic material having a pore-size of from 50 µm to 150 µm, is used to resolve the crude extract from spinach into a brown fraction (A), a yellow fraction (B) and an orange fraction (C).

The orange fraction, C, can be extracted with water and undergo an additional chromatographic separation: in this case, Sephadex G-10 (Reg.Trade Mark), which is dextran that has been cross-linked with epichlorohydrin and has a pore-size of from 40 µm to 120 µm, is used as the chromatographic column-packing material.

Additionally, thin-layer chromatography is used to separate a yellow fraction from the orange fraction C aforementioned.

The Sephadex (Reg.Trade Mark) materials are described in Gel Filtration Theory and Practice by the manufacturers, Farmacia, pages 1-64, to which reference is invited for further details.

The Applicants have isolated several different active antioxidant fractions: they can be used either individually, or in various combinations: it has been ascertained that several combined fractions have exhibited a synergistic antioxidation effect.

The relative amount of the brown, the orange and the yellow fractions can be varied to give optimum results. Generally, any two fractions may be used on a wide range of weight ratios, from 1:99 to 99:1 based on the total weight of the combined fractions.

If desired, more than two fractions may be combined at any desired ratio.

When fatty alimentary substances are to be protected aganst oxidative degradation according to the teachings of this invention, the antioxidant effective amount generally ranges from 0,001% to 1%, and preferably from 0,005% to 0,1% on a weight basis relative to the total weight of the fatty foodstuff plus the antioxidant.

The selection of the concentration of the antioxidant depends on the nature of the fatty substance and the intensity of the oxidation-preventing efficiency which is to be attained.

Alimentary fatty substances which contain solid fats or oils, such as fatty acid esters or free saturated or unsaturated fatty acids, can be protected against degradative oxidation using the water soluble antioxidants extracted and purified according to the teachings of this invention.

The fatty acids are well known substances and are listed, for example, in Noller, Textbook of Organic Chemistry, 2nd Edn., 108-113 (1958).

Examples of fatty alimentary substances are soybean oil, maize oil, cottonseed oil, olive oil, butter, the various margarines, dairy products, milk cream, a few frozen vegetables, soups, fried foods and others.

In addition, foods which contain compounds prone to free-radical oxidation can be protected with the antioxidants prepared according to the teachings of this invention: examples of foods prone to free-radical oxidation are, for example, those which contain flavones, carotenoids, and tocopherol, to mention the most common ones.

It has also been ascertained that the antioxidants prepared according to the teachings of this invention are stable to high temperatures, such as to contact with boiling water for 30 min, have a long stability at room temperature and are definitely nontoxic when ingested.

The general procedure for isolating the water-soluble antioxidants referred to above will now be described and a few practical examples will be given, the invention being also graphically illustrated by the accompanying drawings, wherein:
FIG.1 shows a graphical comparison of the antioxidant effect of a composition made according to this invention with the conventional butylated hydroxytoluene (BHT);
FIG.2 shows an infrared curve of the antioxidant fraction Aₜ as isolated from Trifolium alexandrinum (clover):
FIG.3 shows an infrared curve of the antioxidant fraction Bₜ isolated from clover;
FIG.4 shows an infrared curve of the antioxidant fraction Cₜ isolated from clover, and
FIG.5 shows an infrared curve of the antioxidant fraction A₁ₜ, isolated from clover.

To illustrate the extraction and purification procedure in general terms, a procedure which can be used for obtaining antioxidant water-soluble fractions from the common spinach will be described hereinafter.

Other details can be obtained from the copending EP-A-201 956 from which the present application has been divided out.

Leaves from Spinacia oleracea were homogenized with distilled water at 25°C at a weight:volume ratio of 2:1 (g/ml) in a Waring blender for 5 min. The resulting homogenate was filtered through cheesecloth and then centrifuged at 15.000 times the gravitational field for 10 min., and the supernatant was collected and lyophilized (freeze-dried).

The isolation and purification of antioxidant fractions from the crude homogenate was made by gel-filtration followed by preparative TLC (thin-layer chromatography) or HPLC (high pressure liquid chromatography). 1 g of the lyophilized powder of the crude homogenate was dissolved in 5 ml of distilled water and, after centrifuging at 20.000 times the gravitational field during 10 min, the supernatant was applied to a Sephadex G-25 column (40 cm by 2,5 cm), equilibrated and eluted with distilled water.

Fractions of 5 ml each were collected, and each was tested for antioxidant activity.

The active coloured antioxidant fractions (A, brown-coloured, B, yellow coloured and C, orange-coloured) were pooled, and the lyophilized fraction C was further purified. The lyophilized material of fraction C was dissolved in distilled water to form a 20% solution (weight/volume), centrifuged at 20.000 times the gravitational field during 10 min, whereafter the supernatant was chromatographed on a Sephadex G-10 column (40cm by 2,5 cm) and equilibrated with distilled water.

Fractions C₁ and C₂ were collected separately and lyophilized as aforesaid.

Lyophilized fraction C₁ was dissolved in a minimum amount of distilled water, applied to 0,2 mm-silica gel plates (DC-Karten SIF, Riedel-Dollaen AG, sleeze - Hanover) and developed in aqueous ethanol (30:60 volume/volume).

The active fraction was identified by its pale yellow colour and was extracted from the silica gel plates and lyophilized.

A further purification was carried out using small size DEAE cellulose.

The fraction identified above as A was dissolved in water and passed through a 5 cm by 1 cm column packed with small size DEAE cellulose which had been equilibrated with previously acidified water(0,2N HCl, pH from 5 to 6).

Thereafter, the column was first washed with approximately 50 ml of water and thereafter with 50 ml of aqueous HCl (pH 4). The column was eluted with a solution of HCl (pH 2,0) and the eluted material was recovered in powder form by evaporation in a vacuo: this fraction was identified as fraction A₁.

The powder was dissolved in water at a concentration of 20 µg/ml and passed through a high pressure liquid chromatography silica-60 column (250 mm by 4 mm) with a 90:10 (volume:volume) solution of water:acetonitrile applied at a rate of flow of 0,5 ml/min.

A fraction was obtained, which had a retention fraction at 5,4 nm (UV-absorption spectrum).

Said A₁ fraction analyzed:
For C₈H₁₀O₆N:
Found: C=42.02% ; H=4,80% ; N=6,38% ; 0=40,09%.

An alternative column packing might be Ecteola (Reg.-Trade Mark), a commercial condensation product of cellulose with epichlorohydrin and triethanolamine, having a capacity of from 0,3 meq/g to 0,4 meq/g, and a particle size of from 0,05 mm to 0,2 mm.

### EXAMPLE 1

The crude antioxidant (fractions A, B and C as defined above) was added to linoleic acid to form a mixture containing 20 ml of 7,5 . 10⁻³ of linoleic acid in 0,2 M aqueous sodium phosphate buffer, containing 0,25% by weight of Tween 20 (Reg.Trade Mark) and 1 mg of the crude antioxidant in question. Controls were run, which contained the buffer plus the Tween 20 but no antioxidant, as well as a sample of linoleic acid with 1 mg of BHT (butylated hydroxytoluene) and the same dispersant system. The mixture was kept at 30°C and the optical density was measured using the ferric thiocyanate method described by Koch et al. in Arch.Biochem.Biophys., 78, 165 (1959).

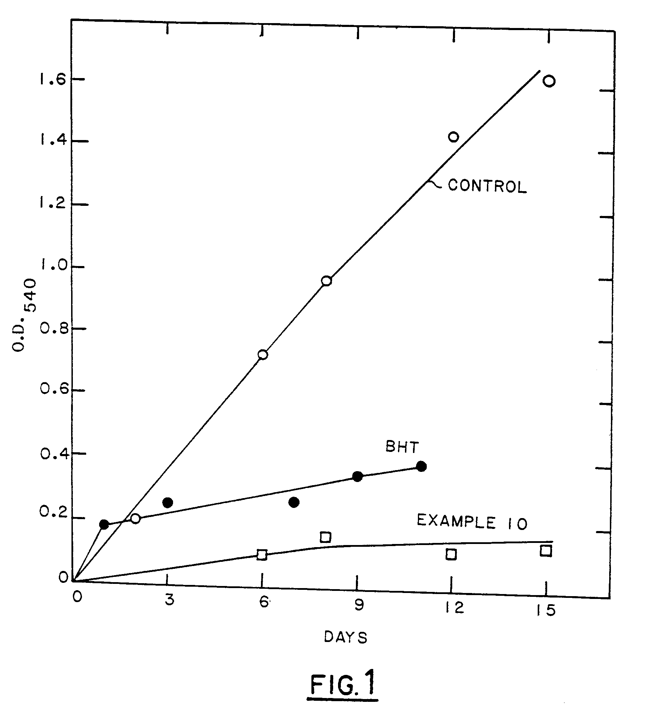

The results of these tests are depicted in Fig.1 of the accompanying drawings, and show that the antioxidant system of this invention is more effective than BHT in preventing the oxidation of the linoleic acid.

### EXAMPLE 2

A procedure similar to that described for spinach in the introductory portion of this description was adopted to isolate antioxidant fractions from Trifolium alexandrinum, a species of clover. The crude extract was separated on Sephadex G-25 (Reg.Trade Mark) to give fractions Aₜ, Bₜ and Cₜ, wherein the suffix t indicates its origin from clover.

Fraction Aₜ was purified on Ecteola (Reg.Trade Mark) to give a fraction, Aₜ₁. Fraction Cₜ was resolved on Sephadex G-10 (Reg.Trade Mark) to give fractions C₁ₜ and C₂ₜ.

Fraction C₁ₜ was further resolved by dissolving it in a minimum amount of water, applying the resultant solution to 0,2 mm-silica gel plates and developing in 30:60 (volume:volume) aqueous ethanol, to give sub-fractions labelled TLC-1 and TLC-2.

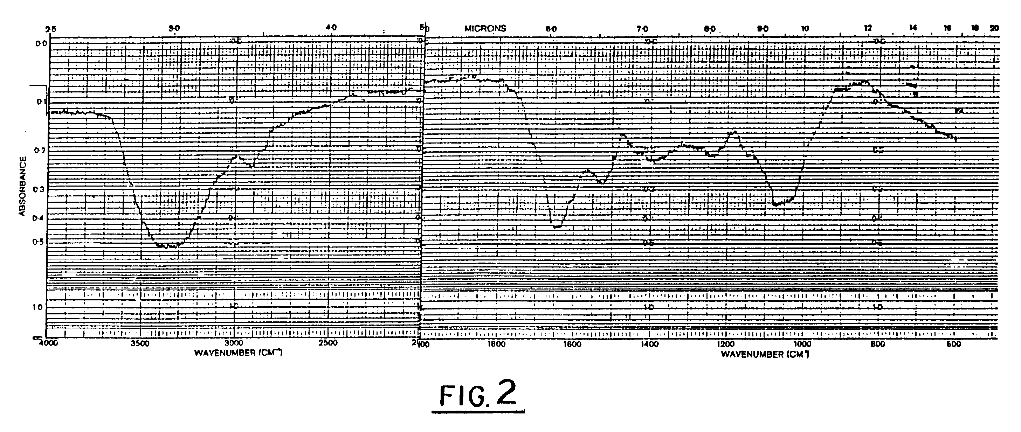

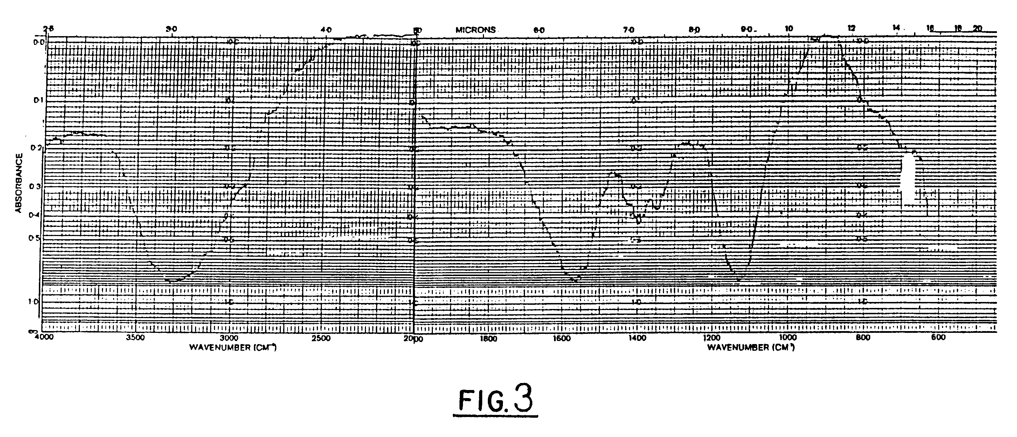

The following data were obtained:
Fraction Aₜ : see the infrared spectrum shown in FIG.2.
Fraction Bₜ : see the infrared spectrum of FIG.3.

This fraction shows strong and broad bands at 3300, 1560 and 1130 cm⁻¹, a medium band at 1400 cm⁻¹, and weak bands at 1350 and 1430 cm⁻¹.

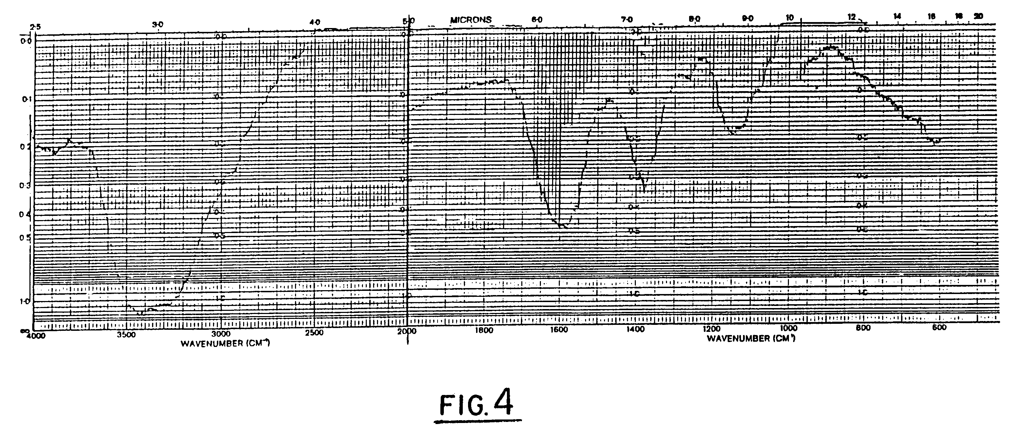

Fraction Cₜ: see the infrared spectrum of FIG.4.

The fraction shows a broad band at 1370 cm⁻¹, and strong bands at 1600, 1380 and 1150 cm⁻¹.

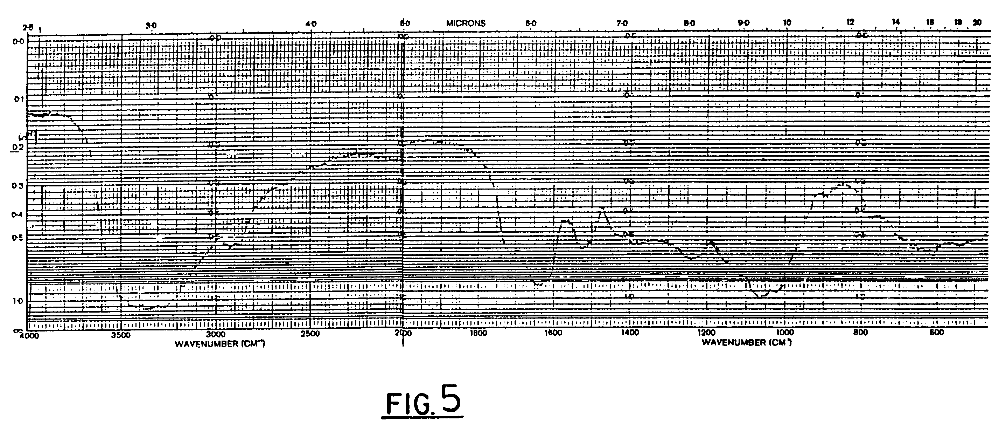

Fraction A₁ₜ : see the infrared spectrum in FIG.5.

Certain of the foregoing fractions (0,2 mg in each case) derived from clover were tested as antioxidants in a system which contained linoleic acid as the substrate, and the lipoxygenase enzyme as the catalyst. Oxygen absorption was monitored using an oxygen monitor according to Grossman and Zakut, Methods of Biochemical Analysis (D.Glick, Publisher), 25, 303-329 (1979).

The results are tabulated hereunder:

| **Inhibition of Lipid Peroxidation by Antioxidant Fractions from clover** | |
|---|---|
| **Fraction** | **%Inhibition** |
| crude extract | 20 |
| Aₜ | 9 |
| Bₜ | 16 |
| Cₜ | 30 |
| TLC-1 | 42 |
| TLC-3 | 46 |

### EXAMPLE 3

A number of Alga samples were homogenized with distilled water, and an extract was prepared according to the procedure described above for Spinacia oleracea.

The crude homogenate was centrifuged and the supernatant was collected and then dried by lyophilization.

The dried crude extracts were tested as antioxidants in a system which contained linoleic acid as the substrate and the lipoxygenase enzyme as the catalyst.

Oxygen consumption was monitored in the same way as indicated in Example 2 above.

The results are tabulated hereunder:

| **Inhibition of Lipid Peroxidation by Antioxidants extracted from Alga species** | |
|---|---|
| **Alga species** | **%Inhibition** |
| Spirulina | 30 |
| Nicractinium | 27 |
| Synicoccus | 30 |
| Navicola | 42 |
| Euglena | 35 |
| Red alga | 35 |

## Claims (Claims for the following Contracting State(s): BE, FR, DE, GB, IT, LU, NL, SE, CH, LI)

1. An oxidation-resistant alimentary fatty composition comprising an alimentary fatty substance, and an active fat-oxidation preventing agent, obtained from vegetable aerial tissues of a plant selected from the genera Spinacia, Trifolium, Medicago, Nicotiana, Alga, Allium, Zea and Penicillaria, characterised in that the active agent is at least one of the coloured antioxidant active fractions which are chromatographically separated, by purification with dextran cross-linked with epichlorohydrin and having a pore size of from 40 µm to 150 µm as the chromatographic column packing, from an aqueous vegetable extract obtained at a temperature of from 4°C to 100°C, preferably at 25 °C, by comminuting in the presence of water, of said vegetable tissues.

2. A composition according to claim 1, wherein the alimentary fatty substance contains a fatty acid ester, a saturated free fatty acid, or an unsaturated free fatty acid or other free-radical oxidative compound.

3. A composition according to claim 1, wherein the alimentary fatty substance is linoleic acid.

4. A composition according to claim 1, wherein said active fat-oxidation preventing agent is the orange fraction whch is chromatographically separated from the supernatant obtained by aqueous extraction of the plant tissue concerned with dextran, which has been cross-linked with epichlorohydrin and has a pore-size of from 50 µm to 150 µm, as the chromatographic column packing.

5. A composition according to claim 1, wherein said active fat-oxidation-preventing agent is the brown fraction which is chromatographically separated from the supernatant obtained by aqueous extraction of the plant tissue concerned from the supernatant obtained by aqueous extraction of the plant tissue concerned using dextran, which has been cross-linked with epichlorohydrin and has a pore-size of from 40 µm to 120 µm, as the chromatographic column packing.

6. A composition according to claim 1, wherein said active fat-oxidation-preventing agent is the yellow fraction which is chromatographically separated from the supernatant obtained by aqueous extraction of the plant tissue concerned using dextran, which has been cross-linked with epichlorohydrin and has a pore size of from 50 µm to 150 µm, as the chromatographic column packing.

7. A composition according to claim 1, wherein said active fat-oxidation-preventing agent is a mixture of at least two of the orange, yellow and brown fractions which have been chromatographically separated from the supernatant obtained by aqueous extraction of the plant tissue concerned using dextran, which has been cross-linked with epichlorohydrin and has a pore-size of from 40 um to 120 um, as the chromatographic column packing.

8. A composition according to claim 1, wherein said active fat-oxidation-preventing agent is any of the fractions chromatographically separated from the aqueous extract of the aerial tissues of Trifolium alexandrinum using dextran, which has been cross-linked with epichlorohydrin and has a pore-size of from 50 µm to 150 µm,as the chromatographic column packing.

9. A composition according to claim 8, wherein said active fat-oxidation-preventing agent is the fraction which is obtained by further chromatographically purifying the yellow fraction, obtained from the purification of the crude extract with dextran which has been cross-linked with epichlorohydrin and has a pore size of from 50 µm to 150 µm, with dextran which has been cross-linked with epichlorohydrin and has a pore-size of from 40 um to 120 um, a dark brown fraction and a yellow orange fraction being thus obtained, and resolving said dark brown fraction by dissolving it in water, applying the resultant aqueous solution to silica gel plates and developing in aqueous ethanol.

10. A composition according to claim 8, wherein said active fat-oxidation-preventing agent is selected from the chromatographic fractions having the following infrared spectral characteristics:
(1) strong and broad bands at 3300, 1560 and 1130 cm⁻¹, medium band at 1400 cm⁻¹, weak bands and 1350 and 1430 cm⁻¹;
(2) broad band at 1370 cm⁻¹, strong bands at 1600, 1380 and 1150 cm⁻¹.

## Claims (Claims for the following Contracting State(s): AT)

1. A process for preparing an oxidation-resistant alimentary fatty composition, characterised by comprising the step of combining an alimentary fatty substance and an active fat-oxidation preventing agent, obtained from vegetable aerial tissues of a plant selected from the genera Spinacia, Trifolium, Medicago, Nicotiana, Alga, Allium, Zea and Penicillaria, said active agent being at least one of the coloured antioxidant active fractions which are chromatographically separated, by purification with dextran cross-linked with epichlorohydrin and having a pore size of from 40 µm to 150 µm as the chromatographic column packing, from an aqueous vegetable extract obtained at a temperature of from 4°C to 100°C, preferably at 25 °C, by comminuting in the presence of water, of said vegetable tissues.

2. A process according to claim 1, wherein the alimentary fatty substance contains a fatty acid ester, a saturated free fatty acid, or an unsaturated free fatty acid or other free-radical oxidative compound.

3. A process according to claim 1, wherein the alimentary fatty substance is linoleic acid.

4. A process according to claim 1, wherein said active fat-oxidation preventing agent is the orange fraction whch is chromatographically separated from the supernatant obtained by aqueous extraction of the plant tissue concerned with dextran, which has been cross-linked with epichlorohydrin and has a pore-size of from 50 µm to 150 µm, as the chromatographic column packing.

5. A process according to claim 1, wherein said active fat-oxidation-preventing agent is the brown fraction which is chromatographically separated from the supernatant obtained by aqueous extraction of the plant tissue concerned from the supernatant obtained by aqueous extraction of the plant tissue concerned using dextran, which has been cross-linked with epichlorohydrin and has a pore-size of from 40 µm to 120 µm, as the chromatographic column packing.

6. A process according to claim 1, wherein said active fat-oxidation-preventing agent is the yellow fraction which is chromatographically separated from the supernatant obtained by aqueous extraction of the plant tissue concerned using dextran, which has been cross-linked with epichlorohydrin and has a pore size of from 50 µm to 150 µm, as the chromatographic column packing.

7. A process according to claim 1, wherein said active fat-oxidation-preventing agent is a mixture of at least two of the orange, yellow and brown fractions which have been chromatographically separated from the supernatant obtained by aqueous extraction of the plant tissue concerned using dextran, which has been cross-linked with epichlorohydrin and has a pore-size of from 40 µm to 120 µm, as the chromatographic column packing.

8. A process according to claim 1, wherein said active fat-oxidation-preventing agent is any of the fractions chromatographically separated from the aqueous extract of the aërial tissues of Trifolium alexandrinum using dextran, which has been cross-linked with epichlorohydrin and has a pore-size of from 50 µm to 150 µm, as the chromatographic column packing.

9. A process according to claim 8, wherein said active fat-oxidation-preventing agent is the fraction which is obtained by further chromatographically purifying the yellow fraction, obtained from the purification of the crude extract with dextran which has been cross-linked with epichlorohydrin and has a pore size of from 50 µm to 150 µm, with dextran which has been cross-linked with epichlorohydrin and has a pore-size of from 40 µm to 120 µm, a dark brown fraction and a yellow orange fraction being thus obtained, and resolving said dark brown fraction by dissolving it in water, applying the resultant aqueous solution to silica gel plates and developing in aqueous ethanol.

10. A process according to claim 8, wherein said active fat-oxidation-preventing agent is selected from the chromatographic fractions having the following infrared spectral characteristics:
(1) strong and broad bands at 3300, 1560 and 1130 cm⁻¹, medium band at 1400 cm⁻¹, weak bands and 1350 and 1430 cm⁻¹;
(2) broad band at 1370 cm⁻¹, strong bands at 1600, 1380 and 1150 cm⁻¹.

## Patentansprüche (Patentansprüche für folgende(n) Vertragsstaat(en): BE, FR, DE, GB, IT, LU, NL, SE, CH, LI)

1. Oxidationsbeständige Speisefettzusammensetzung, umfassend eine Speisefettsubstanz und ein aktives, die Fettoxidation verhinderndes Mittel, erhalten aus pflanzlichen Luftgeweben einer Pflanze, ausgewählt aus den Arten Spinacia, Trifolium, Medicago, Nicotiana, Alga, Allium, Zea und Penicillaria, dadurch **gekennzeichnet,** daß das aktive Mittel mindestens eine der farbigen, als Antioxidans aktiven Fraktionen ist, die chromatographisch durch Reinigung mit Dextran, das mit Epichlorhydrin vernetzt ist und als chromatographische Säulenfüllung eine Porengröße von 40 µm bis 150 µm aufweist, aus einem wäßrigen, pflanzlichen Extrakt, erhalten bei einer Temperatur von 4°C bis 100°C, vorzugsweise bei 25°C, durch Zerkleinern der genannten pflanzlichen Gewebe in Gegenwart von Wasser, getrennt werden.

2. Zusammensetzung nach Anspruch 1, dadurch **gekennzeichnet,** daß die Speisefettsubstanz einen Fettsäureester, eine gesättigte freie Fettsäure oder eine ungesättigte freie Fettsäure oder eine andere frei-radikalische oxidative Verbindung enthält.

3. Zusammensetzung nach Anspruch 1, dadurch **gekennzeichnet,** daß die Speisefettsubstanz Linolsäure ist.

4. Zusammensetzung nach Anspruch 1, dadurch **gekennzeichnet,** daß das aktive, die Fettoxidation verhindernde Mittel die orangefarbene Fraktion ist, die chromatographisch von dem Überstand abgetrennt worden ist, der durch wäßrige Extraktion des entsprechenden Pflanzengewebes mit Dextran erhalten worden ist, das mit Epichlorhydrin vernetzt worden ist und als chromatographische Säulenfüllung eine Porengröße von 50 µm bis 150 µm aufweist.

5. Zusammensetzung nach Anspruch 1, dadurch **gekennzeichnet,** daß das aktive, die Fettoxidation verhindernde Mittel die braune Fraktion ist, die chromatographisch von dem Überstand abgetrennt worden ist, der durch wäßrige Extraktion des entsprechenden Pflanzengewebes, von dem Überstand erhalten worden ist, der durch wäßrige Extraktion des entsprechenden Pflanzengewebes mit Dextran erhalten worden ist, das mit Epichlorhydrin vernetzt worden ist und als chromatographische Säulenfüllung eine Porengröße von 40 µm bis 120 µm aufweist.

6. Zusammensetzung nach Anspruch 1, dadurch **gekennzeichnet,** daß das aktive, die Fettoxidation verhindernde Mittel die gelbe Fraktion ist, die chromatographisch von dem Überstand abgetrennt worden ist, der durch wäßrige Extraktion des entsprechenden Pflanzengewebes mit Dextran erhalten worden ist, das mit Epichlorhydrin vernetzt worden ist und als chromatographische Säulenfüllung eine Porengröße von 50 µm bis 150 µm aufweist.

7. Zusammensetzung nach Anspruch 1, dadurch **gekennzeichnet,** daß das aktive, die Fettoxidation verhindernde Mittel ein Gemisch von mindestens zwei der orangefarbigen, gelben und braunen Fraktionen ist, die chromatographisch von dem Überstand abgetrennt worden sind, der durch wäßrige Extraktion des entsprechenden Pflanzengewebes mit Dextran erhalten worden ist, das mit Epichlorhydrin vernetzt worden ist und als chromatographische Säulenfüllung eine Porengröße von 40 µm bis 120 µm aufweist.

8. Zusammensetzung nach Anspruch 1, dadurch **gekennzeichnet,** daß das aktive, die Fettoxidation verhindernde Mittel eine der Fraktionen ist, die chromatographisch von dem wäßrigen Extrakt der Luftgewebe von Trifolium alexandrinum unter Verwendung von Dextran, das mit Epichlorhydrin vernetzt worden ist und eine Porengröße von 50 µm bis 150 µm aufweist, als chromatographische Säulenfüllung, abgetrennt worden sind.

9. Zusammensetzung nach Anspruch 8, dadurch **gekennzeichnet,** daß das aktive, die Fettoxidation verhindernde Mittel die Fraktion ist, die durch weitere chromatographische Reinigung der gelben Fraktion, die aus der Reinigung des Rohextrakts mit Dextran, das mit Epichlorhydrin vernetzt worden ist und eine Porengröße von 50 µm bis 150 µm aufweist, mit Dextran, das mit Epichlorhydrin vernetzt worden ist und eine Porengröße von 40 µm bis 120 µm aufweist, wobei auf diese Weise eine dunkelbraune Fraktion und eine gelborangefarbige Fraktion erhalten werden, und Trennung der dunkelbraunen Fraktion durch Auflösen in Wasser, Aufbringen der resultierenden wäßrigen Lösung auf Kieselgelplatten und Entwikkeln in wäßrigem Ethanol, erhalten worden ist.

10. Zusammensetzung nach Anspruch 8, dadurch **gekennzeichnet,** daß das aktive, die Fettoxidation verhindernde Mittel aus den chromatographischen Fraktionen ausgewählt ist, die die folgenden Infrarot-spektroskopischen Eigenschaften aufweisen:
(1) starke und breite Banden bei 3300, 1560 und 1130 cm⁻¹, eine mittlere Bande bei 1400 cm⁻¹ und schwache Banden bei 1350 und 1430 cm⁻¹;
(2) breite Bande bei 1370 cm⁻¹, starke Banden bei 1600, 1380 und 1150 cm⁻¹.

## Patentansprüche (Patentansprüche für folgende(n) Vertragsstaat(en): AT)

1. Verfahren zur Herstellung einer oxidationsbeständigen Speisefettzusammensetzung, dadurch **gekennzeichnet,** daß es die Stufe der Kombination einer Speisefettsubstanz und eines aktiven, die Fettoxidation verhindernden Mittels umfaßt, das aus pflanzlichen Luftgeweben einer Pflanze, ausgewählt aus den Arten Spinacia, Trifolium, Medicago, Nicotiana, Alga, Allium, Zea und Penicillaria erhalten worden ist, wobei das aktive Mittel mindestens eine der farbigen, als Antioxidans aktiven Fraktionen ist, die chromatographisch durch Reinigung mit Dextran, das mit Epichlorhydrin vernetzt worden ist und als chromatographisches Säulenmaterial eine Porengröße von 40 µm bis 150 µm aufweist, aus einem wäßrigen, pflanzlichen Extrakt, erhalten bei einer Temperatur von 4°C bis 100°C, vorzugsweise bei 25°C, durch Zerkleinern der genannten pflanzlichen Gewebe in Gegenwart von Wasser, getrennt worden sind.

2. Verfahren nach Anspruch 1, dadurch **gekennzeichnet,** daß die Speisefettsubstanz einen Fettsäureester, eine gesättigte freie Fettsäure oder eine ungesättigte freie Fettsäure oder eine andere frei-radikalische oxidative Verbindung enthält.

3. Verfahren nach Anspruch 1, dadurch **gekennzeichnet,** daß die Speisefettsubstanz Linolsäure ist.

4. Verfahren nach Anspruch 1, dadurch **gekennzeichnet,** daß das aktive, die Fettoxidation verhindernde Mittel die orangefarbene Fraktion ist, die chromatographisch von dem Überstand abgetrennt worden ist, der durch wäßrige Extraktion des entsprechenden Pflanzengewebes mit Dextran erhalten worden ist, das mit Epichlorhydrin vernetzt worden ist und als chromatographische Säulenfüllung eine Porengröße von 50 µm bis 150 µm aufweist.

5. Verfahren nach Anspruch 1, dadurch **gekennzeichnet,** daß das aktive, die Fettoxidation verhindernde Mittel die braune Fraktion ist, die chromatographisch von dem Überstand abgetrennt worden ist, der durch wäßrige Extraktion des entsprechenden Pflanzengewebes, von dem Überstand erhalten worden ist, der durch wäßrige Extraktion des entsprechenden Pflanzengewebes mit Dextran erhalten worden ist, das mit Epichlorhydrin vernetzt worden ist und als chromatographische Säulenfüllung eine Porengröße von 40 µm bis 120 µm aufweist.

6. Verfahren nach Anspruch 1, dadurch **gekennzeichnet,** daß das aktive, die Fettoxidation verhindernde Mittel die gelbe Fraktion ist, die chromatographisch von dem Überstand abgetrennt worden ist, der durch wäßrige Extraktion des entsprechenden Pflanzengewebes mit Dextran erhalten worden ist, das mit Epichlorhydrin vernetzt worden ist und als chromatographische Säulenfüllung eine Porengröße von 50 µm bis 150 µm aufweist.

7. Verfahren nach Anspruch 1, dadurch **gekennzeichnet,** daß das aktive, die Fettoxidation verhindernde Mittel ein Gemisch von mindestens zwei der orangefarbigen, gelben und braunen Fraktionen ist, die chromatographisch von dem Überstand abgetrennt worden sind, der durch wäßrige Extraktion des entsprechenden Pflanzengewebes mit Dextran erhalten worden ist, das mit Epichlorhydrin vernetzt worden ist und als chromatographische Säulenfüllung eine Porengröße von 40 µm bis 120 µm aufweist.

8. Verfahren nach Anspruch 1, dadurch **gekennzeichnet,** daß das aktive, die Fettoxidation verhindernde Mittel eine der Fraktionen ist, die chromatographisch von dem wäßrigen Extrakt der Luftgewebe von Trifolium alexandrinum unter Verwendung von Dextran, das mit Epichlorhydrin vernetzt worden ist und eine Porengröße von 50 µm bis 150 µm aufweist, als chromatographische Säulenfüllung, abgetrennt worden sind.

9. Verfahren nach Anspruch 8, dadurch **gekennzeichnet,** daß das aktive, die Fettoxidation verhindernde Mittel die Fraktion ist, die durch weitere chromatographische Reinigung der gelben Fraktion, die aus der Reinigung des Rohextrakts mit Dextran, das mit Epichlorhydrin vernetzt worden ist und eine Porengröße von 50 µm bis 150 µm aufweist, mit Dextran, das mit Epichlorhydrin vernetzt worden ist und eine Porengröße von 40 µm bis 120 µm aufweist, wobei auf diese Weise eine dunkelbraune Fraktion und eine gelborangefarbige Fraktion erhalten werden, und Trennung der dunkelbraunen Fraktion durch Auflösen in Wasser, Aufbringen der resultierenden wäßrigen Lösung auf Kieselgelplatten und Entwickeln in wäßrigem Ethanol, erhalten worden ist.

10. Verfahren nach Anspruch 8, dadurch **gekennzeichnet,** daß das aktive, die Fettoxidation verhindernde Mittel aus den chromatographischen Fraktionen ausgewählt ist, die die folgenden Infrarot-spektroskopischen Eigenschaften aufweisen:
(1) starke und breite Banden bei 3300, 1560 und 1130 cm⁻¹, eine mittlere Bande bei 1400 cm⁻¹ und schwache Banden bei 1350 und 1430 cm⁻¹;
(2) breite Bande bei 1370 cm⁻¹, starke Banden bei 1600, 1380 und 1150 cm⁻¹.

## Revendications (Revendications pour l'(les) Etat(s) contractant(s) suivant(s): BE, FR, DE, GB, IT, LU, NL, SE, CH, LI)

1. Composition alimentaire grasse résistant à l'oxydation, comprenant une substance alimentaire grasse, et un agent actif empêchant l'oxydation de la graisse, obtenu à partir de tissus aériens végétaux d'une plante choisie parmi les genres Spinacia, Trifolium, Medicago, Nicotiana, Alga, Allium, Zea et Penicillaria, caractérisée en ce que l'agent actif est au moins l'une des fractions actives antioxydantes colorées qui sont séparées par chromatographie, par purification avec du dextrane réticulé à l'épichlorhydrine et ayant une taille de pores de 40 µm à 150 µm pour l'empilement de la colonne de chromatographie, à partir d'un extrait végétal aqueux obtenu à une température de 4°C à 100°C, de préférence à 25°C, par fragmentation desdits tissus végétaux en présence d'eau.

2. Composition selon la revendication 1, dans laquelle la substance alimentaire grasse contient un ester d'acide gras, un acide gras libre saturé, ou un acide gras libre insaturé, ou un autre composé oxydant à radical libre.

3. Composition selon la revendication 1, dans laquelle la substance alimentaire grasse est l'acide linoléique.

4. Composition selon la revendication 1, dans laquelle ledit agent actif empêchant l'oxydation de la graisse est la fraction orange qui est séparée par chromatographie à partir du surnageant obtenu par extraction aqueuse du tissu de plante concerné, avec du dextrane qui a été réticulé avec de l'épichlorhydrine et qui a une taille de pores de 50 µm à 150 µm pour l'empilement de la colonne de chromatographie.

5. Composition selon la revendication 1, dans laquelle ledit agent actif empêchant l'oxydation de la graisse est la fraction brune qui est séparée par chromatographie à partir du surnageant obtenu par extraction aqueuse du tissu de plante concerné, avec du dextrane qui a été réticulé avec de l'épichlorhydrine et qui a une taille de pores de 40 µm à 120 µm pour l'empilement de la colonne de chromatographie.

6. Composition selon la revendication 1, dans laquelle ledit agent actif empêchant l'oxydation de la graisse est la fraction jaune qui est séparée par chromatographie à partir du surnageant obtenu par extraction aqueuse du tissu de plante concerné, avec du dextrane qui a été réticulé avec de l'épichlorhydrine et qui a une taille de pores de 50 µm à 150 µm pour l'empilement de la colonne de chromatographie.

7. Composition selon la revendication 1, dans laquelle ledit agent actif empêchant l'oxydation de la graisse est un mélange d'au moins deux des fractions orange, jaune et brune qui ont été séparées par chromatographie à partir du surnageant obtenu par extraction aqueuse du tissu de plante concerné, avec du dextrane qui a été réticulé avec de l'épichlorhydrine et une taille de pores de 40 µm à 120 µm pour l'empilement de la colonne de chromatographie.

8. Composition selon la revendication 1, dans laquelle ledit agent actif empêchant l'oxydation de la graisse est l'une quelconque des fractions qui ont été séparées par chromatographie à partir de l'extrait aqueux de tissus aériens de Trifolium alexandrinum, à l'aide de dextrane qui a été réticulé avec de l'épichlorhydrine et qui a une taille de pores de 50 µm à 150 µm pour l'empilement de la colonne de chromatographie.

9. Composition selon la revendication 8, dans laquelle ledit agent actif empêchant l'oxydation de la graisse est la fraction qui est obtenue après une nouvelle purification par chromatographie de la fraction jaune, cette dernière ayant été obtenue par purification de l'extrait brut avec du dextrane qui a été réticulé avec de l'épichlorhydrine et qui a une taille de pores de 50 µm à 150 µm, avec du dextrane qui a été réticulé avec de l'épichlorhydrine et qui a une taille de pores de 40 µm à 120 µm, une fraction brun foncé et une fraction jaune orangé étant ainsi obtenues, par redissolution de ladite fraction brun foncé dans l'eau, application de la solution aqueuse obtenue sur des plaques de gel de silice et développement dans de l'éthanol aqueux.

10. Composition selon la revendication 8, dans laquelle ledit agent actif empêchant l'oxydation de la graisse est choisi parmi les fractions obtenues par chromatographie ayant les caractéristiques spectrales en infrarouge suivantes :
(1) bandes larges et intenses à 3300, 1560 et 1130 cm⁻¹, bande intermédiaire à 1400 cm⁻¹, bandes faibles à 1350 et 1430 cm⁻¹;
(2) bande large à 1370 cm⁻¹, bandes intenses à 1600, 1380 et 1150 cm⁻¹.

## Revendications (Revendications pour l'(les) Etat(s) contractant(s) suivant(s): AT)

1. Procédé de préparation d'une composition alimentaire grasse résistant à l'oxydation, caractérisé par le fait de comprendre l'étape consistant à combiner une substance alimentaire grasse et un agent actif empêchant l'oxydation de la graisse, obtenu à partir de tissus aériens végétaux d'une plante choisie parmi les genres Spinacia, Trifolium, Medicago, Nicotiana, Alga, Allium, Zea et Penicillaria, caractérisé en ce que l'agent actif est au moins l'une des fractions actives antioxydantes colorées qui sont séparées par chromatographie, par purification avec du dextrane réticulé à l'épichlorhydrine et ayant une taille de pores de 40 µm à 150 µm pour l'empilement de la colonne de chromatographie, à partir d'un extrait végétal aqueux obtenu à une température de 4°C à 100°C, de préférence à 25°C, par fragmentation desdits tissus végétaux en présence d'eau.

2. Procédé selon la revendication 1, dans lequel la substance alimentaire grasse contient un ester d'acide gras, un acide gras libre saturé, ou un acide gras libre insaturé, ou un autre composé oxydant à radical libre.

3. Procédé selon la revendication 1, dans lequel la substance alimentaire grasse est l'acide linoléique.

4. Procédé selon la revendication 1, dans lequel ledit agent actif empêchant l'oxydation de la graisse est la fraction orange qui est séparée par chromatographie à partir du surnageant obtenu par extraction aqueuse du tissu de plante concerné, avec du dextrane qui a été réticulé avec de l'épichlorhydrine et qui a une taille de pores de 50 µm à 150 µm pour l'empilement de la colonne de chromatographie.

5. Procédé selon la revendication 1, dans laquelle ledit agent actif empêchant l'oxydation de la graisse est la fraction brune qui est séparée par chromatographie à partir du surnageant obtenu par extraction aqueuse du tissu de plante concerné, avec du dextrane qui a été réticulé avec de l'épichlorhydrine et qui a une taille de pores de 40 µm à 120 µm pour l'empilement de la colonne de chromatographie.

6. Procédé selon la revendication 1, dans lequel ledit agent actif empêchant l'oxydation de la graisse est la fraction jaune qui est séparée par chromatographie à partir du surnageant obtenu par extraction aqueuse du tissu de plante concerné, avec du dextrane qui a été réticulé avec de l'épichlorhydrine et qui a une taille de pores de 50 µm à 150 µm pour l'empilement de la colonne de chromatographie.

7. Procédé selon la revendication 1, dans lequel ledit agent actif empêchant l'oxydation de la graisse est un mélange d'au moins deux des fractions orange, jaune et brune qui ont été séparées par chromatographie à partir du surnageant obtenu par extraction aqueuse du tissu de plante concerné, avec du dextrane qui a été réticulé avec de l'épichlorhydrine et qui a une taille de pores de 40 µm à 120 µm pour l'empilement de la colonne de chromatographie.

8. Procédé selon la revendication 1, dans lequel ledit agent actif empêchant l'oxydation de la graisse est l'une quelconque des fractions qui ont été séparées par chromatographie à partir de l'extrait aqueux de tissus aériens de Trifolium alexandrinum, à l'aide de dextrane qui a été réticulé avec de l'épichlorhydrine et qui a une taille de pores de 50 µm à 150 µm pour l'empilement de la colonne de chromatographie.

9. Procédé selon la revendication 8, dans lequel ledit agent actif empêchant l'oxydation de la graisse est la fraction qui est obtenue après une nouvelle purification par chromatographie de la fraction jaune, cette dernière ayant été obtenue par purification de l'extrait brut avec du dextrane qui a été réticulé avec de l'épichlorhydrine et une taille de pores de 50 µm à 150 µm, avec du dextrane qui a été réticulé avec de l'épichlorhydrine et qui a une taille de pores de 40 µm à 120 µm, une fraction brun foncé et une fraction jaune orangé étant ainsi obtenues, par redissolution de ladite fraction brun foncé dans l'eau, application de la solution aqueuse obtenue sur des plaques de gel de silice et développement dans de l'éthanol aqueux.

10. Procédé selon la revendication 8, dans lequel ledit agent actif empêchant l'oxydation de la graisse est choisi parmi les fractions ayant les caractéristiques spectrales en infrarouge suivantes :
(1) bandes larges et intenses à 3300, 1560 et 1130 cm⁻¹, bande intermédiaire à 1400 cm⁻¹, bandes faibles à 1350 et 1430 cm⁻¹ ;
(2) bande large à 1370 cm⁻¹, bandes intenses à 1600, 1380 et 1150 cm⁻¹.
